# FASCICULE DE BREVET EUROPEEN

(11) **EP 0 981 350 B1**
(45) Date de publication et mention de la délivrance du brevet: **04.10.2001**
(21) Numéro de dépôt: 98910810.5
(22) Date de dépôt: 20.02.1998
(51) Int. Cl.: A61K 31/59

(54) **UTILISATION DU 9,10-SECOCHOLESTA-5,7,10(19)-TRIEN-1,3-OL, OU ALFACALCIDOL**
VERWENDUNG VON 9,10-SECOCHOLESTA-5,7,10(19)-TRIEN-1,3-OL (ALFACALCIDOL)
USE OF 9,10-SECOCHOLESTA-5,7,10(19)-TRIENE-1,3-DIOL, OR ALFACALCIDOL

(30) Priorité: 20.02.1997 FR 9702247
(43) Date de publication de la demande: 01.03.2000
(73) Titulaire: TROUILLAS, Paul, F-69006 Lyon (FR)
(72) Inventeur: TROUILLAS, Paul, F-69006 Lyon (FR)
(74) Mandataire: Maureau, Philippe
(86) Numéro de dépôt international: FR9800347
(87) Numéro de publication internationale: WO9836754

(56) Documents cités:
- WO-A-96/00074
- COLSTON K W ET AL: "POSSIBLE ROLE FOR VITAMIN D IN CONTROLLING BREAST CANCER CELL PROLIFERATION" LANCET THE, vol. 1, 28 janvier 1989, pages 188-191, XP002044141
- SATO ET AL.: "Antitumor Effect of 1 alpha-Hydroxyvitamin D3" TOHOKU J. EXP. MED., vol. 138, no. 4, 1982, pages 445--446, XP002065408
- BINDERUP L.: "VITAMIN D ANALOGUES: NEW REGULATORS OF CANCER CELL GROWTH AND DIFFERENTIATION" BIOORG. MED. CHEM. LETT., vol. 3, no. 9, 1993, pages 1891-1896, XP002065409
- HONMA Y ET AL: "1 ALPHA,25-DIHYDROXYVITAMIN D3 AND 1 ALPHA-HYDROXYVITAMIN D3 PROLONG SURVIVAL TIME OF MICE INOCULATED WITH MYELOID LEUKEMIA CELLS" PROCEEDINGS OF THE NATIONAL ACADEMY OF SCIENCES OF USA, vol. 80, janvier 1983, pages 201-204, XP002044142
- SATO ET AL.: "Effect of 1 alpha-hydroxyvitamin D3 on metastasis of rat ascites hepatome k-231" BR. J. CANCER, vol. 50, no. 1, 1984, pages 123-125, XP002065410
- COLSTON K W ET AL: "EFFECTS OF SYNTHETIC VITAMIN D ANALOGUES ON BREAST CANCER CELL PROLIFERATION IN VIVO AND IN VITRO" BIOCHEMICAL PHARMACOLOGY, vol. 44, no. 4, 18 août 1992, pages 693-702, XP002044143
- IINO Y ET AL: "1ALPHA-HYDROXYVITAMIN D3, HYPERCALCEMIA, AND GROWTH SUPPRESSION OF 7,12-DIMETHYLBENZ[A]ANTHRACENE-INDUCED RAT MAMMARY TUMORS" BREAST CANCER RESEARCH AND TREATMENT, vol. 22, 1992, pages 133-140, XP002044144
- HICKISH T ET AL: "THE EFFECT OF 1,25-DIHYDROXYVITAMIN D3 ON LYMPHOMA CELL LINES AND EXPRESSION OF VITAMIN D RECEPTOR IN LYMPHOMA" BRITISH JOURNAL OF CANCER, vol. 68, no. 4, octobre 1993, pages 668-672, XP002044145
- KAWAURA A ET AL: "1ALPHA-HYDROXYVITAMIN D3 SUPPRESSES COLONIC TUMORIGENESIS INDUCED BY REPETITIVE INTRARECTAL INJECTION OF N-METHYL-N-NITROSOUREA IN RATS" CANCER LETTERS, vol. 55, no. 2, 3 décembre 1990, pages 149-152, XP002044146
- SKOWRONSKI R J ET AL: "ACTIONS OF VITAMIN D3 ANALOGS ON HUMAN PROSTATE CANCER CELL LINES: COMPARISON WITH 1,25-DIHYDROXYVITAMIN D3" ENDOCRINOLOGY, vol. 138, no. 1, 1995, pages 20-26, XP002058578
- SRIVASTAVA M D ET AL: "DIFFERENTIATION OF NEOPLASTIC CELLS TOWARD NORMAL INDUCED BY VITAMIN D3 DERIVATIVES" RESEARCH COMMUNICATIONS IN CHEMICAL PATHOLOGY AND PHARMACOLOGY, vol. 83, no. 2, février 1994, pages 115-123, XP000618445

## Description

La présente invention concerne l'utilisation du 9,10-secocholesta-5,7,10(19)-trien-1,3-ol, ou alfacalcidol, pour la fabrication d'un médicament destiné au traitement et/ou à la prévention des tumeurs solides de l'homme, notamment le cancer du poumon et les cancers de l'encéphale.

La vitamine D3, ou cholécalciférol, est une hormone essentielle pour maintenir chez l'homme une concentration constante en calcium. Cette vitamine D peut être formée dans la peau , à partir du 7-déhydrocholestérol, sous l'action de la lumière ultra-violette, ou être ingérée par la nourriture. L'hormone dérivée de la vitamine D3, métaboliquement active, se forme par deux hydroxylations : dans le foie en position 25 (calcifédiol), puis dans le rein en position 1 (calcitriol). L'hormone-vitamine D3, stimule l'absorption de calcium et de phosphate au niveau de l'intestin. Par suite de l'élévation des concentrations de calcium et de phosphate, la tendance à la cristallisation dans l'os sous forme d'hydroxyapatite augmente. En cas de carence en vitamine D, la minéralisation osseuse est insuffisante et ceci a comme conséquence le rachitisme et l'ostéomalacie. En général, la thérapie consiste à administrer de la vitamine D, on peut aussi utiliser le calcifédiol chez des sujets ayant des troubles hépatiques ou le calcitriol chez les personnes porteuses d'une affection rénale. Le calcitriol présente une activité supérieure, pour réguler la concentration en calcium, par rapport au calcifédiol et à la vitamine D3. En cas de surdosage, il se produit une hypercalcémie avec dépôt de sels de calcium dans les tissus, principalement les reins et les vaisseaux, induisant une calcinose.

Le 9,10-secocholesta-5,7,10(19)-trien-1,3-ol, ou 1-alpha-hydroxy-cholécalciférol, ou alfacalcidol, est un précurseur synthétique de la vitamine D3. Ce dérivé est transformé dans le foie en calcitriol. Ce dérivé alfacalcidol est connu et est utilisé dans les affections suivantes: ostéodystrophie rénale, rachitisme pseudocarentiel, rachitisme ostéomalacique par hypophosphatémie vitamino-résistante, hypoparathyroidie et pseudo hypoparathyroidie, ainsi qu'en prévention de l'hypocalcémie postparathyroidectomie et hypocalcémie néo natale tardive.

Le brevet US 4,391,802 divulgue l'utilisation de l'alfacalcidol, à des doses de 1 *µ*g/jour pour le traitement de patients atteints de leucémie.

Le document de Colston et al. (" Possible role for vitamine D in controlling breast cancer proliferation ", The Lancet, vol 1, 1989, pages 188-191) divulgue l'efficacité de l'alfacalcidol pour inhiber la prolifération in vitro de lignées cellulaires cancéreuses du sein. Ce document montre que le traitement de rats par l'alfacalcidol, porteurs de tumeur du sein induite chimiquement, engendre des problèmes d'hypercalcémie.

Le document de Colston et al. (" Effects of Synthetic vitamin D analogues on breast cancer cell proliferation in vivo and in vitro ", Biochem. Pharmacol., vol 44, no 4, page 693-702, 1992) divulgue l'utilisation d'un analogue de la vitamine D, le calcipotriol, pour traiter in vivo des rats atteints d'un cancer du sein induit chimiquement. Il est montré par ailleurs que l'alfacalcidol engendre une hypercalcémie chez le rat, phénomène qui va à l'encontre des effets inhibiteurs sur le cancer.

Le document de Lino et al. ((" 1-alphahydroxyvitamin D3, hypercalcemia, and growth suppression of 7,12-dimethylbenz[a]anthracene-induced mammary tumors ", Breast Cancer Res. Treat., vol 22, no 2, page 133-140, 1992) montre que l'alfacalcidol, bien qu'inhibant un cancer du sein induit chez le rat, génère des effets secondaires de perte de poids et d'hypercalcémie.

Au vue de l'art antérieur cité ci-dessus, il apparaît que le calcipotriol soit le seul dérivé potentiellement efficace pour inhiber des cancers du sein induits chez le rat sans provoquer d'hypercalcémie.

La demanderesse a découvert de manière surprenante que l'alfacalcidol pouvait être utilisé chez l'homme pour le traitement et/ou la prévention des cancers solides pendant une période de plusieurs années.

La présente invention a donc pour objet l'utilisation de l'alfacalcidol pour la fabrication d'un médicament destiné au traitement et/ou à la prévention des cancers solides chez l'homme.

Par cancers solides de l'homme, on entend toutes les tumeurs malignes autres qu'hématologiques. Ces cancers solides comprennent notamment les cancers du sein, du poumon, du cerveau, de l'appareil digestif, de l'appareil gynécologique, de l'appareil génital masculin, et les sarcomes.

Par traitement des cancers solides, on entend la disparition des cancers.

Par prévention des cancers, on entend l'empêchement d'une part de l'apparition de nouveaux cancers, et d'autre part de la réapparition d'un cancer en rémission, ou rechute.

Dans un mode de réalisation préféré selon l'invention, l'utilisation de l'alfacalcidol est destinée au traitement à long terme des cancers solides de l'homme.

Dans un autre mode de réalisation préféré selon l'invention, l'utilisation de l'alfacalcidol est destinée à la prévention anti-cancéreuse à l'égard du développement des cancers et/ou la rechute après traitement. On peut également utiliser l'alfacalcidol en adjonction dans la nourriture pour prévenir les apparitions des cancers.

Dans un mode de réalisation très préféré selon l'invention, les cancers solides sont des cancers spontanés.

Par cancers spontanés, on entend selon la présente invention des cancers " naturels ", non induits chimiquement.

La posologie du traitement sera fonction du type de cancer à traiter et de l'importance évolutive du cancer, ou des risques qu'une personne peut présenter à développer un cancer.

Dans un mode de réalisation préféré selon l'invention, la dose efficace est comprise entre 0,25 et 10 *µ*g/jour pour un adulte de 60 kg.

Dans un mode de réalisation très préféré selon l'invention, la dose efficace est comprise entre 1 et 5 *µ*g/jour pour un adulte de 60 kg.

Dans un mode de réalisation encore plus préféré selon l'invention, la dose efficace est de 1 ou 2 *µ*g/jour pour un adulte de 60 kg.

Les exemples suivants démontrent qu'un traitement par l'alfacalcidol, à une dose de 1 ou 2 *µ*g/jour pour un adulte de 60 kg, est efficace sans diète particulière pour traiter les cancers solides.

Les exemples qui suivent démontrent en clinique chez l'homme que le 1-alpha-hydroxy-cholécalciférol permet d'influencer le développement de divers cancers solides, après résultat incomplet de la chimiothérapie conventionnelle et/ou de la radiothérapie, ou en l'absence de ces thérapeutiques, à savoir:
- régression remarquable de cancers présumés gravissimes,
- amélioration de l'évolution clinique, biologique et générale,
- mise en évidence de la régression radiologique tant au scanner qu'en Imagerie par Résonance Magnétique (IRM) des cancers solides,
- caractère progressif de l'effet, s'étalant sur plusieurs mois, commençant et se manifestant à la suite de l'instauration du traitement,
- simultanément et après le traitement, le patient ne présente pas d'hypercalcémie, d'hyperphosphorémie, d'hypermagnésiémie, d'hypercalciurie et le taux de phosphatases alcalines est normal.

La composition pharmaceutique contenant le 1-alpha-hydroxy-cholécalciférol se présente de préférence sous une forme galénique convenable pour l'administration orale et, par exemple, peut être administrée sous forme de comprimés, capsules, gélules et pilules. Il est également possible d'utiliser une forme galénique liquide pour une administration par voie parentérale intramusculaire ou sous cutanée.

Les exemples qui suivent présentent des études cliniques réalisées en pratique hospitalière et ambulatoire chez des patients porteurs de cancers solides, ayant des lésions identifiées en l'absence de tout autre traitement, ou après une subsistance du cancer malgré des traitements ayant comporté une chirurgie seule, une chimiothérapie seule, une radiothérapie seule, ou les combinaisons chirurgie-radiothérapie, chirurgie-chimiothérapie, ou encore la combinaison chirurgie-chimiothérapie-radiothérapie.

Vingt patients ont été traités à long terme avec de l'alfacalcidol à dose de 1 et 2 *µ*g/jour pour un adulte de 60 kg. Les effets thérapeutiques sont observés à long terme et peuvent être détectés dès 3 mois après l'instauration du traitement. on observe alors une diminution significative du volume du cancer, qui peut être suivie par des moyens radiologiques, tandis que l'état clinique s'améliore.

L'administration clinique pendant un an et plus a pu être effectuée dans certaines observations sans complication aucune, ni anomalie particulière de la calcémie, de la phosphorémie, de la magnésiémie, des phosphatases alcalines et de la calciurie.

Les exemples qui suivent illustrent l'invention mais ne doivent pas être considérés comme une limitation de la portée des revendications ci-jointes. En l'occurence, il est tout a fait envisageable de traiter des cancers d'enfants, voire de nourrissons avec l'alfacalcidol.

### Exemple 1 :

Une patiente âgée de 73 ans, atteinte de carcinome pulmonaire, présentait à la bronchoscopie une tumeur envahissant la partie postérieur de la bronche souche G. La biopsie montrait un néoplasme indifférencié, possiblement d'origine neuro-endocrine. L'IRM montrait une invasion de la colonne dorsale par une volumineuse tumeur en D3-D4-D5-D6.

Cette patiente a été traitée par radiothérapie sur la colonne dorsale, puis par 6 cures de chimiothérapie avec du carboplatine-VP16.

Les examens à trois mois ont montré que la tumeur était toujours présente, tant au niveau pulmonaire qu'au niveau des métastases de la colonne vertébrale. Il a également été diagnostiqué des métastases hépatiques.

La patiente a alors été traité avec 1 *µ*g/jour de 1-alpha-hydroxy-calciférol.

Après trois mois de ce traitement, il est observé une régression de la tumeur à la bronchoscopie et à l'IRM, et on observe une sémiologie clinique améliorée.

Après un an de traitement, on observe une radiographie thoracique normalisée, l'IRM dorsale présente des images cicatricielles, et le scanner abdominal ne montre plus de métastases hépatiques.

Après trois ans de traitement, on observe toujours une radiographie thoracique normalisée, l'IRM dorsale et médullaire est alors pratiquement normale et il n'existe plus aucun symptôme de tumeur.

La patiente peut alors être considérée comme guérie sur le plan carcinologique.

Le traitement est alors poursuivi à titre de prévention anti-cancéreuse.

Simultanément, il est important de noter que la patiente, après ces années de traitement, ne présente pas d'hypercalcémie, d'hyperphosphorémie, d'hypermagnésiémie, d'hypercalciurie et le taux de phosphatases alcalines est normal.

### Exemple 2 :

Un patient de 50 ans atteint d'un astrocytome grade III de localisation frontale postérieure, associé à une maladie de Fahr, a subi une radiothérapie de 60 grays et une chimiothérapie toutes les 6 semaines avec du féniposide (VM 26) et de l'omustine (CCNU).

A l'issue du processus thérapeutique, le scanner et l'IRM montrent la persistance d'une volumineuse tumeur frontale postérieure droite prenant le contraste de l'iode au scanner et du gadolinium à l'IRM. Le patient est alors encore hémiplégique gauche et présente de nombreuses crises Bravais-Jacksoniennes du côté gauche.

On a alors traité le patient avec 1 *µ*g/jour de 1-alpha-hydroxy-calciférol pendant trois ans.

Pendant ces trois années, on va observer une évolution clinique progressive. Il apparaît en premier lieu une diminution de la comitialité Bravais-Jacksonienne gauche, puis une disparition totale de celle-ci. On observe ensuite une récupération progressive du déficit, faisant passer le patient de la petite voiture à des capacités de marche autonome. Les IRM répétés tous les 6 mois au cours du traitement montrent la diminution progressive de la prise de gadolinium, puis sa disparition totale après les trois années de traitement.

Le traitement est alors poursuivi à titre de prévention anti-cancéreuse.

Simultanément, après ces années de traitement, le patient ne présente pas d'hypercalcémie, d'hyperphosphorémie, d'hypermagnésiémie, d'hypercalciurie et le taux de phosphatases alcalines est normal.

Le patient a repris son activité professionnelle tout en continuant son traitement.

### Exemple 3 :

Un patient âgé de 55 ans, atteint d'un glioblastome grade IV occipital gauche, a en premier lieu été opéré de sa tumeur. L'exérèse n'ayant pas été complète, le patient subit une radiothérapie de 60 grays et une chimiothérapie comportant 6 cures, de VM 26 et CCNU.

A l'issue de la chimiothérapie, on observait nettement la tumeur au scanner et à l'IRM.

On a alors traité le patient avec 1 *µ*g/jour d'1-alpha-hydroxy-calciférol pendant deux ans.

On observe alors un silence symptomatique total, tandis que, sur les IRM avec gadolinium, la prise de contraste tend à disparaître.

Après trois ans de traitement, la tumeur a encore diminué et ne présente plus qu'une imprégnation de la taille d'un petit pois. L'état clinique est alors excellent.

Simultanément, après ces années de traitement, le patient ne présente pas d'hypercalcémie, d'hyperphosphorémie, d'hypermagnésiémie, d'hypercalciurie et le taux de phosphatases alcalines est normal.

### Exemple 4 :

Une patiente âgée de 87 ans, atteinte d'un glioblastome grade IV de localisation temporale gauche, présentait une aphasie de Wernicke complète. On observait une volumineuse tumeur prenant le contraste.

La patiente a subi une chimiothérapie par VM26-CCNU.

A l'issue de la chimiothérapie, l'énorme tumeur persiste au scanner, avec une importante prise de contraste.

On a alors traité la patiente avec 1 *µ*g/jour d'1-alpha-hydroxy-calciférol pendant un an.

On observe alors, au cours du traitement, par des scanners répétés, la diminution remarquable de la prise de contraste et la diminution du volume globale de la tumeur.

Après un an de traitement, l'aphasie de Wernicke a beaucoup diminué. On observe alors au scanner une prise de contraste moindre et un volume de la tumeur moindre.

Simultanément, après ces années de traitement, la patiente ne présente pas d'hypercalcémie, d'hyperphosphorémie, d'hypermagnésiémie, d'hypercalciurie et le taux de phosphatases alcalines est normal.

### Exemple 5 :

Un patient âgé de 53 ans, atteint d'une tumeur maligne du lobe gauche latéro-aortique, et ne présentant aucune perfusion pulmonaire à la scintigraphie pulmonaire, a subi une pneumonectomie gauche intra-péricardique. A l'histologie, il a été diagnostiqué un carcinome malpighien et des ganglions inter-trachéobronchiques. Le bilan d'extension était à T2 N3 M0.

Le patient a alors subi une radiothérapie de 66 grays en champs complexes pendant 7 semaines, avec chimiothérapie hebdomadaire avec du cisplatine et de la navelbine.

Les examens après le protocole de chimio-radiothérapie ont montré des métastases osseuses ischiopubiennes droites et zygomatiques bilatérales, un nodule périphérique de la pyramide basale, et un aspect de sténose de la bronche de Nelson dont la biopsie a démontré le caractère néoplasique.

On a alors traité le patient avec 1 *µ*g/jour d'1-alpha-hydroxy-calciférol pendant deux ans.

Au cours du traitement, on observe une amélioration progressive des principaux éléments pathologiques : désinfiltration de la sténose de la bronche de Nelson, diminution des volumes des métastases osseuses sur les radiographies.

Après les deux ans de traitement, l'état clinique du patient est stabilisé.

Simultanément, après ces années de traitement, la patiente ne présente pas d'hypercalcémie, d'hyperphosphorémie, d'hypermagnésiémie, d'hypercalciurie et le taux de phosphatases alcalines est normal.

### Exemple 6 :

Un patient âgé de 67 ans était atteint d'un glioblastome grade IV de localisation temporale droite.

Il a subi une exérèse chirurgicale incomplète et une radiothérapie de 60 grays sur la tumeur pendant 6 semaines.

A l'issue du processus thérapeutique, il présentait la persistance d'une image temporale droite profonde à l'IRM, avec prise de gadolinium.

Le patient de 62 Kg est alors traité avec 2 *µ*g/jours d'alfacalcidol sur une période de deux ans.

On observe la diminution progressive de la taille de l'image IRM prenant le gadolinium.

L'état clinique du patient est stable.

Simultanément, après ces années de traitement, le patient ne présente pas d'hypercalcémie, d'hyperphosphorémie, d'hypermagnésiémie, d'hypercalciurie et le taux de phosphatases alcalines est normal.

## Revendications

1. Utilisation du [9,10-secocholesta-5,7,10 (19)-trien-1,3-ol], ou alfacalcidol, pour la fabrication d'un médicament destiné au traitement et/ou à la prevention des cancers solides non hématologiques de l'homme, sur la base d'une dose efficace comprise entre 0,25 et 10 µg / jour d'alfacalcidol pour un adulte de 60 kg.

2. Utilisation selon la revendication 1, sur la base d'une dose efficace comprise entre 1 et 5 µg/jour pour un adulte de 60 kg.

3. Utilisation selon la revendication 2, sur la base d'une dose efficace de 1 ou 2 µg / jour pour un adulte de 60 kg.

4. Utilisation du [9,10-secocholesta-5,7,10 (19)-trien-1,3-ol], ou alfacalcidol, pour la fabrication d'un médicament destiné au traitement et/ou la prévention des cancers solides chez l'homme selon laquelle l'alfacalcidol est administrable en quantité suffisante pour obtenir la régression dudit cancer solide.

5. Utilisation selon la revendication 4 **caractérisée en ce que** l'alfacalcidol est administrable en quantité adaptée pour ne pas engendrer une autre affection appartenant au groupe constitué par l'hypercalcémie, l'hyperphosphorémie, l'hypermagnésiémie et l'hypercalciurie.

6. Utilisation selon l'une quelconque des revendications précédentes, pour le traitement et/ou la prévention de cancers spontanés.

7. Utilisation du [9,10-secocholesta-5,7,10 (19)-trien-1,3-ol], ou alfacalcidol, pour la fabrication d'un médicament destiné au traitement et/ou la prévention des cancers de l'encéphale.

8. Utilisation selon la revendication 7, pour le traitement et/ou la prévention des cancers gliomateux.

9. Utilisation selon l'une quelconque des revendications 7 ou 8, pour le traitement et/ou la prévention de cancers spontanés.

## Patentansprüche

1. Verwendung von [9,10-Secocholesta-5,7,10(19)-trien-1,3-ol] bzw. Alfacalcidol zur Herstellung eines Arzneimittels zur Behandlung und/oder zur Prävention von nicht hämatologischen menschlichen soliden Karzinomen auf Basis einer wirksamen Dosis von 0,25 bis 10 *µ*g pro Tag Alfacalcidol für einen Erwachsenen von 60 kg.

2. Verwendung nach Anspruch 1 auf Basis einer wirksamen Dosis von 1 bis 5 *µ* g pro Tag für einen Erwachsenen von 60 kg.

3. Verwendung nach Anspruch 2 auf Basis einer wirksamen Dosis von 1 oder 2 *µ*g pro Tag für einen Erwachsenen von 60 kg.

4. Verwendung von [9,10-Secocholesta-5,7,10(19)-trien-1,3-ol] bzw. Alfacalcidol zur Herstellung eines Arzneimittels zur Behandlung und/oder zur Prävention von menschlichen soliden Karzinomen, bei der das Alfacalcidol in einer ausreichenden Menge verabreicht wird, um eine Regression des soliden Karzinoms zu erreichen.

5. Verwendung nach Anspruch 4, **dadurch gekennzeichnet, daß** das Alfacalcidol in einer Menge verabreichbar ist, die so angelegt ist, **daß** kein anderes Leiden hervorgerufen wird, das der Gruppe zugehörig ist gebildet aus Hyperkalziämie, Hyperphosphorämie, Hypermagnesiämie und Hyperkalziurie.

6. Verwendung nach einem der vorhergehenden Ansprüche zur Behandlung und/oder zur Prävention von spontanen Karzinomen.

7. Verwendung von [9,10-Secocholesta-5,7,10 (19)-trien-1,3-ol] bzw. Alfacalcidol zur Herstellung eines Arzneimittels zur Behandlung und/oder zur Prävention von enzephalen Karzinomen.

8. Verwendung nach Anspruch 7 zur Behandlung und/oder zur Prävention von gliomatösen Karzinomen.

9. Verwendung nach einem der Ansprüche 7 oder 8 zur Behandlung und/oder zur Prävention von spontanen Karzinomen.

## Claims

1. Use of [9,10-secocholesta-5,7,10(19)-trien-1,3-ol], or alfacalcidol, for the manufacture of a medicament intended for the treatment and/or prevention of nonhaematological solid cancers in humans, on the basis of an effective dose of between 0.25 and 10 µg/day of alfacalcidol for an adult of 60 kg.

2. Use according to Claim 1, on the basis of an effective dose of between 1 and 5 µg/day for an adult of 60 kg.

3. Use according to Claim 2, on the basis of an effective dose of 1 or 2 µg/day for an adult of 60 kg.

4. Use of [9,10-secocholesta-5,7,10(19)-trien-1,3-ol], or alfacalcidol, for the manufacture of a medicament intended for the treatment and/or prevention of solid cancers in humans according to which the alfacalcidol is administrable in a quantity sufficient to obtain the regression of the said solid cancer.

5. Use according to Claim 4, **characterized in that** the alfacalcidol is administrable in an appropriate quantity so as not to cause another condition belonging to the group consisting of hypercalcaemia, hyperphosphataemia, hypermagnesiaemia and hypercalciuria.

6. Use according to any one of the preceding claims, for the treatment and/or prevention of spontaneous cancers.

7. Use of [9,10-secocholesta-5,7,10(19)-trien-1,3-ol], or alfacalcidol, for the manufacture of a medicament intended for the treatment and/or prevention of cancers of the encephalon.

8. Use according to Claim 7, for the treatment and/or prevention of gliomatous cancers.

9. Use according to either of Claims 7 and 8, for the treatment and/or prevention of spontaneous cancers.
